# EUROPEAN PATENT SPECIFICATION

(11) **EP 3 592 329 B1**
(45) Date of publication and mention of the grant of the patent: **18.02.2026**
(21) Application number: 18708416.5
(22) Date of filing: 05.03.2018
(51) Int. Cl.: A61K 8/36, A61K 8/49, A61Q 19/02

(54) **USE OF COMPATIBLE SOLUTES**
VERWENDUNG VON KOMPATIBLEN GELÖSTEN STOFFEN
UTILISATION DE SOLUTÉS COMPATIBLES

(30) Priority: 06.03.2017 EP 17159311
(43) Date of publication of application: 15.01.2020
(73) Proprietor: SUSONITY Commercial GmbH, 64579 Gernsheim (DE)
(72) Inventor: HEIDER, Lilia, 64579 Gernsheim (DE); HAMASAKI, Takeshi, Tokyo 107-0062 (JP); MASAKI, Hitoshi, Hachiouji-shi Tokyo 192-0982 (JP)
(74) Representative: Weckenbrock, Matthias
(86) International application number: PCT/EP2018/055251
(87) International publication number: WO 2018/162368

(56) References cited:
- EP-A2- 2 246 037
- WO-A1-00/07559
- WO-A1-2012/014901
- WO-A1-2016/169373
- WO-A1-2016/183203
- CA-A1- 2 958 258
- DE-A1- 10 002 725
- JP-A- 2011 148 715
- US-A1- 2004 253 332
- US-A1- 2005 201 955
- US-A1- 2005 232 953
- US-A1- 2008 305 059
- US-A1- 2009 041 866
- US-A1- 2009 227 523
- US-A1- 2012 114 576
- US-A1- 2015 017 269
- US-A1- 2015 118 165
- US-A1- 2015 118 172
- US-A1- 2016 120 781

## Description

The present invention relates to the use of at least one compatible solute as defined in claims for countering age-related skin color change or for the suppression and/or reduction of protein carbonylation and/or for the suppression and/or reduction of the accumulation of carbonylated proteins in the stratum corneum as defined in claims.

Skin color is one of the major factors that determine our esthetic perception of skin. It is well known that with ageing skin color may change to yellow-dark and become uneven. A few potential causes leading to skin color change have been proposed. Within this regard oxidized proteins such as advanced glycation end products and carbonylated proteins which are end-products of lipid peroxidation may cause the skin color change to yellow-dark in elder people (Ogura et al. 2011, Journal of Dermatological Science 64, 45-52). Recently, it was found that the enlargement of skin pores may also be involved in unevenness of skin color. The microscopic observation of skin revealed darkness around skin pores, which is different from follicular plugging. Such darker colored areas might also be resulting in uneven skin color. It was reported that the darkening around skin pores is not caused by melanin, but by the accumulation of carbonylated proteins in the stratum corneum around the skin pores (Akane Sasaki, August 2016, "The accumulation of carbonylated proteins causes darkness around skin pores", The 17th Annual Meeting of the Society for Photoaging Research).

Surprisingly, it was now found that compatible solutes are capable of solving this problem.

The use of compatible solutes for various cosmetic and pharmaceutical purposes is already known.

For example, WO 94/15923 describes that (S)-1,4,5,6-tetrahydro-2-methyl-4-pyrimidinecarboxylic acid or (S,S)-1,4,5,6-tetrahydro-5-hydroxy-2-methyl-4-pyrimidinecarboxylic acid can be used for the preparation of a cosmetic composition or a medicament, for example for the treatment of skin diseases. Furthermore, DE4342560 describes the use of ectoin and ectoin derivatives as moisturisers in cosmetic products. These products are suitable, for example, for the care of aged, dry or irritated skin.

Furthermore, DE19933466 describes that ectoin and derivatives, such as hydroxyectoin, can be employed as free-radical scavengers in cosmetic and dermatological compositions. The compositions can be used for the treatment and/or prophylaxis of skin ageing caused by oxidative stress and of inflammatory reactions.

Further applications of ectoin and ectoin derivatives in cosmetic formulations are described, for example, in WO 00/07558, WO 00/07559, WO 00/07560, and US 7981899, such as, for example, the care and prophylaxis of dry and/or flaky skin, protection of the human skin against dryness and/or high salt concentrations, protection of cells, proteins and/or biomembranes of the human skin, protection of the microflora of the human skin, stabilisation of the skin barrier and protection and stabilisation of nucleic acids of human skin cells.

Protein carbonylation is occurring in the whole body, including the skin, and can be triggered by reactive aldehyde compounds. Such can be reactive aldehydes of exogenous origin or endogeneously formed aldehydes, e.g. resulting from oxidative degradation of polyunsaturated fatty acids, which are ubiquitously found in the body. The resulting aldehydes are, e.g. acrolein and 4-hydroxynonenal. Such aldehydes can for example be formed in the degradation of skin surface sebum. Carbonylated proteins can thus be found in the stratum corneum, particularly in the upper portion of the sun-exposed area. It is known that carbonylated proteins produced in the upper dermis, rather than melanin pigmentation, are mostly responsible for the development of the yellowish skin color alteration associated with photo-ageing (Ogura et al. 2011, Journal of Dermatological Science 64, 45-52). Such alteration of skin color to a yellowish or darker color very often occurs around skin pores which is due to the fact that carbonylated proteins tend to accumulate around pores (Akane Sasaki, August 2016, "The accumulation of carbonylated proteins causes darkness around skin pores", The 17th Annual Meeting of the Society for Photoaging Research).

Within this regard compatible solutes were found to be capable of lightening skin pores.

It is used for lightening of skin pores and/or for the reduction of the number of yellow or dark colored skin pores in human skin.

Skin pores are small openings present at the skin surface. In general, they are believed to pour out noxious and waste substances present in the body. In such a concept, they correspond to the ostia of the sweat gland apparatus. However, the term pore also applies to visible microtopographic features at the skin surface corresponding to enlarged openings of pilosebaceous follicles. They appear as empty funnel-shaped structures or, by contrast, as cornified cylindrical plugs corresponding to comedones.

It is known that compatible solutes are capable of minimizing oxidative stress within skin due to the formation of heat shock proteins and other protective properties. It was further found that compatible solutes are capable of reducing the formation of carbonylated proteins within the stratum corneum. In a further embodiment the invention therefore relates to the use of at least one compatible solute and/or its physiologically acceptable salt for the suppression and/or reduction of protein carbonylation and/or for the suppression and/or reduction of the accumulation of carbonylated proteins in the stratum corneum, in particular around skin pores.

The term "suppression and or reduction of protein carbonylation" relates to any reduction in protein carbonylation activity which is based on the action of the specific compounds according to the invention. The term "suppression and/or reduction of the accumulation of carbonylated proteins in the stratum corneum" relates to any reduction of the accumaltion of carbonylated proteins which is based on the action of the specific compounds according to the invention.

The stratum corneum is the outermost layer of the epidermis, consisting of dead cells (corneocytes). This layer is composed of 15 to 20 layers of flattened cells with no nuclei and cell organelles. Their cytoplasm shows filamentous keratin. These corneocytes are embedded in a lipid matrix composed of ceramides, cholesterol and fatty acids.

The stratum corneum functions to form a barrier to protect underlying tissue from infection, dehydration, chemicals and mechanical stress. Desquamation, the process of cell shedding from the surface of the stratum corneum, balances proliferating keratinocytes that form in the stratum basale. These cells migrate through the epidermis towards the surface in a journey that takes approximately 14 days.

The above-mentioned use of the compounds can take place in in-vitro or in-vivo models. The susceptibility of a particular cell to treatment with the compounds of claim 1 can be determined by testing in vitro. For testing in vitro, cultivated cells from a biopsy sample can be used. The use in vitro takes place, in particular, on samples of human species which are suffering from age-induced skin color change.

The testing of a plurality of specific compounds enables the selection of the active compound which appears the most suitable for the treatment.

The use of the compatible solutes as defined above is typically topical and a non-therapeutic or cosmetic use.

According to a common definition compatible solutes are stress protection substances from extremely halophilic and halotolerant eubacteria, which accumulate them in large amounts through biosynthesis or effective transport mechanisms. These osmotically active substances prevent liquid outflow into the medium (drying out) and owe their name to the fact that they do not impair cell metabolism, even in high cytoplasmic concentration, i.e. are compatible with metabolism (according to: E.A. Galinski, M. Stein, B. Amendt, M. Kinder Comp. Biochem. Physiol., 117 (3) (1997) 357-365).

Ectoin and ectoin derivatives are low-molecular-weight, cyclic amino acid derivatives which can be isolated from various halophilic microorganisms or prepared synthetically. Both ectoin and hydroxyectoin have the advantage of not reacting with cell metabolism.

The one compatible solute is selected from (S)-1,4,5,6-tetrahydro-2-methyl-4-pyrimidincarboxylic acid (ectoin) and (S,S)-1,4,5,6-tetrahydro-5-hydroxy-2-methyl-4-pyrimidincarboxylic acid (hydoxyectoin).

Most preferred, the at least one compabtible solute is (S)-1,4,5,6-tetrahydro-2-methyl-4-pyrimidincarboxylic acid (ectoin).

Compounds as described in claims in which basic and acidic groups, such as carboxyl or amino groups, are present in equal number form internal salts.

The preparation of the compounds is described in the literature (DE4342560). (S)-1,4,5,6-Tetrahydro-2-methyl-4-pyrimidinecarboxylic acid or (S,S)-1,4,5,6-tetrahydro-5-hydroxy-2-methyl-4-pyrimidinecarboxylic acid can also be obtained by microbiological methods (Severin et al., J. Gen. Microb. 138 (1992) 1629-1638 or EP1409707A).

It is known that compatible solutes are capable of minimizing oxidative stress within skin due to the formation of heat shock proteins and other protective properties. It was however unkown and first described in the present invention that compatible solutes are further capable of reducing the formation of carbonylated proteins. Since carbonylated proteins tend to accumulate around skin pores and are the reason for the skin color change to yellow/ brown in these areas, the reduction of carbonylated protein formation leads to a more uniform skin color and less dark- or yellow-colored pores in the skin. Even skin-color can be restored.

For the purposes of the present invention, the term "agent", "composition" or "formulation" is also used synonymously alongside the term "preparation".

The preparations here are usually preparations which can be applied topically, such as, for example, cosmetic or dermatological formulations or medical products. "Can be applied topically" in the sense of the invention means that the preparation is applied externally and locally, i.e. that the preparation must be suitable, for example, for being able to be applied to the skin. In this case, the preparations comprise a cosmetically, pharmaceutically or dermatologically suitable vehicle and, depending on the desired property profile, optionally further suitable ingredients. The topical preparations are preferably employed as cosmetic or dermatological preparation, particularly preferably as cosmetic preparation. Suitable vehicles and assistants or fillers are described in detail in the following part.

The preparations may include or comprise, essentially consist of or consist of the necessary or optional constituents mentioned above and/or below. All compounds or components which can be used in the preparations are either known and commercially available or can be synthesised by known processes. Further preferred combinations of embodiments are disclosed in the claims.

The compatible solute, as indicated above and also as preferably described, is typically present in the preparation of the invention in an amount of 0.001 to 50% by weight, based on the total weight of the preparation, preferably from 0.01 to 10% by weight and especially preferably from 0.1 to 10% by weight, based on the composition as a whole. The proportion of the said compounds in the composition is very especially preferably from 0.1 to 5% by weight, based on the composition as a whole. The person skilled in the art is presented with absolutely no difficulties in selecting appropriately the amounts depending on the intended effect of the preparation.

Besides the compatible solutes, the compositions may also comprise further cosmetic, dermatological or pharmaceutical active ingredients.

In one embodiment the invention is directed to a formulation comprising at least one compatible solute and/or its physiologically acceptable salt and at least one further skin pore lightening active.

Preferred embodiments of the at least one compatible solute are defined as described above. In one particular preferred embodiment, the at least one compatible solute in the formulation is selected from the group of compounds as defined above.

Further skin pore lightening actives are known to the person skilled in the art and may for example be chosen from alpha hydroxy acids (AHA), such as, for example, citric acid, lactic acid or malic acid, and salicylic acid, vitamin C and its derivatives and emblica.

The further active compound is preferably selected from the group of UV filters, pore-refining agents, antioxidants, vitamins, skin-lightening active compounds, anti-ageing active compounds, anti-inflammatory active compounds, antimicrobial active compounds, active compounds for improving the moisture content of the skin (skin-moisture regulators), anticellulite active compounds, antiwrinkle active compounds, antidandruff active compounds, anti-acne active compounds, deodorants, pigments and self-tanning substances; particularly preferably from the group of UV filters, pore-refining agents, antioxidants, vitamins, skin-lightening active compounds, self-tanning substances, anti-ageing active compounds and anticellulite active compounds.

In one preferred embodiment the formulation further comprises UV filters. In principle, all UV filters are suitable for combination within the preparation according to the invention. Particular preference is given to UV filters whose physiological acceptability has already been demonstrated. There are many proven substances known from the specialist literature both for UVA and also UVB filters. The compounds shown in the following lists should only be regarded as examples. Other UV filters can of course also be used. Preferred preparations may comprise organic UV filters, so-called hydrophilic or lipophilic sun-protection filters, which are effective in the UVA region and/or UVB region and/or IR and/or VIS region (absorbers). These substances can be selected, in particular, from dibenzoylmethane derivatives, p-aminobenzoic acid derivatives, salicylic acid derivatives, β,β-diphenylacrylate derivatives, camphor derivatives, triazine derivatives, cinnamic acid derivatives and polymeric filters and silicone filters, which are described in the application WO 93/04665. Further examples of organic filters are indicated in the patent application EP-A 0 487 404. The said UV filters are usually named below in accordance with INCI nomenclature.

Particularly suitable for a combination are:
Dibenzoylmethane derivatives: 4-isopropyl-dibenzoyl-methane and 4,4'-methoxy-tert-butyl-dibenzoylmethane being described in FR-A-2326405, FR-A-2440933 and EP-A-0114607. 4,4'-Methoxy-tert-butyl-dibenzoylmethane is for example marketed by Merck under the name "Eusolex 9020".

para-Aminobenzoic acid and derivatives thereof: PABA, Ethyl PABA, Ethyl dihydroxypropyl PABA, Ethylhexyl dimethyl PABA, for example marketed by ISP under the name "Escalol 507", Glyceryl PABA, PEG-25 PABA, for example marketed by BASF under the name "Uvinul P25".

Salicylates: Homosalate marketed by Merck under the name "Eusolex HMS"; Ethylhexyl salicylate, for example marketed by Symrise under the name "Neo Heliopan OS"; Dipropylene glycol salicylate, for example marketed by Scher under the name "Dipsal"; TEA salicylate, for example marketed by Symrise under the name "Neo Heliopan TS".

β,β-Diphenylacrylate derivatives: Octocrylene, for example marketed by Merck under the name "Eusolex^{®} OCR"; "Uvinul N539" from BASF; Etocrylene, for example marketed by BASF under the name "Uvinul N35".

Benzophenone derivatives: benzophenone-1, for example marketed under the name "Uvinul 400"; benzophenone-2, for example marketed under the name "Uvinul D50"; benzophenone-3 or oxybenzone, for example marketed under the name "Uvinul M40"; benzophenone-4, for example marketed under the name "Uvinul MS40"; benzophenone-9, for example marketed by BASF under the name "Uvinul DS-49"; benzophenone-5, benzophenone-6, for example marketed by Norquay under the name "Helisorb 11"; benzophenone-8, for example marketed by American Cyanamid under the name "Spectra-Sorb UV-24"; benzophenone-12 n-hexyl 2-(4-diethylamino-2-hydroxybenzoyl) benzoate or 2-hydroxy-4-methoxybenzophenone, marketed by Merck, Darmstadt, under the name Eusolex^{®} 4360.

Benzylidenecamphor derivatives: 3-benzylidenecamphor, for example marketed by Chimex under the name "Mexoryl SD"; 4-methylbenzylidenecamphor, for example marketed by Merck under the name "Eusolex 6300"; benzylidenecamphorsulfonic acid, for example marketed by Chimex under the name "Mexoryl SL"; Camphor benzalkonium methosulfate, for example marketed by Chimex under the name "Mexoryl SO"; terephthalylidene-dicamphorsulfonic acid, for example marketed by Chimex under the name "Mexoryl SX"; polyacrylamidomethylbenzylidenecamphor marketed by Chimex under the name "Mexoryl SW".

Phenylbenzimidazole derivatives: phenylbenzimidazolesulfonic acid, for example marketed by Merck under the name "Eusolex 232"; disodium phenyl dibenzimidazole tetrasulfonate, for example marketed by Symrise under the name "Neo Heliopan AP".

Phenylbenzotriazole derivatives: Drometrizol trisiloxane, for example marketed by Rhodia Chimie under the name "Silatrizole"; Methylenebis(benzotriazolyl)tetramethylbutylphenol in solid form, for example marketed by Fairmount Chemical under the name "MIXXIM BB/100", or in micronised form as an aqueous dispersion, for example marketed by BASF under the name "Tinosorb M".

Triazine derivatives: Ethylhexyltriazone, for example marketed by BASF under the name "Uvinul T150"; Diethylhexylbutamidotriazone, for example marketed by Sigma 3V under the name "Uvasorb HEB"; 2,4,6-tris-(diisobutyl 4'-aminobenzalmalonate)-s-triazine or 2,4,6-Tris(biphenyl)-1,3,5-triazine marketed by BASF as Tinosorb A2B; 2,2'-[6-(4-methoxyphenyl)-1,3,5-triazine-2,4-diyl]bis[5-(2-ethylhexyl)oxy]phenol; marketed by BASF as Tinosorb S; N2,N4-bis[4-[5-(1,1-dimethylpropyl)-2-benzoxazolyl]phenyl]-N6-(2-ethylhexyl)-1,3,5-triazine-2,4,6-triamine marketed as Uvasorb K 2A by Sigma 3V, or trisbiphenyltriazin, marketed as Tinosorb^{®} A2B by BASF.

Anthraniline derivatives: Menthyl anthranilate, for example marketed by Symrise under the name "Neo Heliopan MA".

Imidazole derivatives: ethylhexyldimethoxybenzylidenedioxoimidazoline propionate.

Benzalmalonate derivatives: polyorganosiloxanes containing functional benzalmalonate groups, such as, for example, Polysilicone-15, for example marketed by Hoffmann LaRoche under the name "Parsol SLX".

4,4-Diarylbutadiene derivatives: 1,1-Dicarboxy(2,2'-dimethylpropyl)-4,4-diphenylbutadiene.

Benzoxazole derivatives: 2,4-bis[5-(1-dimethylpropyl)benzoxazol-2-yl(4-phenyl) imino]-6-(2-ethylhexyl)imino-1,3,5-triazine, for example marketed by Sigma 3V under the name Uvasorb K2A, and mixtures comprising this.

Piperazine derivatives, such as, for example, the compound 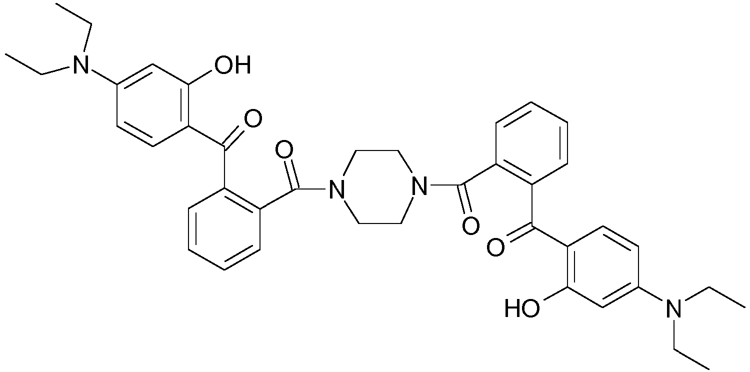 or the UV filters of the following structures

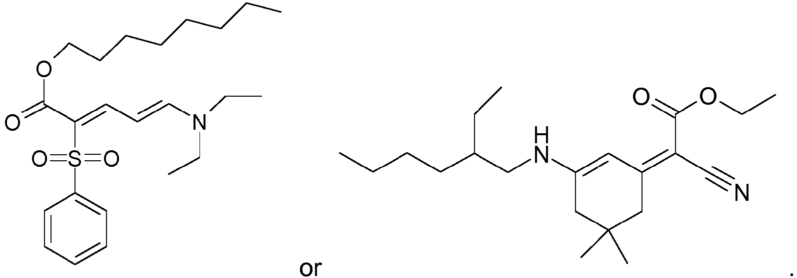

It is also possible to use UV filters based on polysiloxane copolymers having a random distribution in accordance with the following formula, where, for example, a = 1,2; b= 58 and c=2,8:

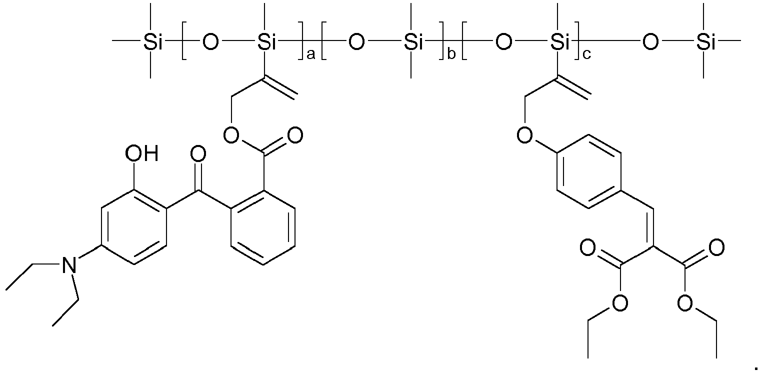

This list of compounds represents examples; it is of course also possible to use other UV filters.

Suitable organic UV-protecting substances can preferably be selected from the following list: Ethylhexyl salicylate, phenylbenzimidazolesulfonic acid, benzophenone-3, benzophenone-4, benzophenone-5, n-Hexyl 2-(4-diethyl-amino-2-hydroxybenzoyl)benzoate, 4-methylbenzylidenecamphor, tere-phthalylidenedicamphorsulfonic acid, disodium phenyldibenzimidazole-tetrasulfonate, methylenebis(benzotriazolyl)tetramethylbutylphenol, Butyl methoxydibenzoylmethane, Ethylhexyltriazone, Diethylhexylbutamidotriazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine and mixtures thereof.

These organic UV filters are generally incorporated into formulations in an amount of 0.01 to 20 per cent by weight, preferably 1 to 10% by weight.

The preparations may comprise further inorganic UV filters, so-called particulate UV filters. These combinations with particulate UV filters are possible both as powder and also as dispersion or paste of the following types.

Preference is given here both to those from the group of the titanium dioxides, such as, for example, coated titanium dioxide (for example Eusolex^{®} T-2000, Eusolex^{®} T-AQUA, Eusolex^{®} T-AVO, Eusolex^{®} T-PRO, Eusolex^{®} T-EASY), zinc oxides (for example Sachtotec^{®}), iron oxides or also cerium oxides and/or zirconium oxides.

Furthermore, combinations with pigmentary titanium dioxide or zinc oxide are also possible, where the particle size of these pigments is greater than or equal to 200 nm, for example Hombitan^{®} FG or Hombitan^{®} FF-Pharma.

It may furthermore be preferred for the preparations to comprise inorganic UV filters which have been aftertreated by conventional methods, as described, for example, in Cosmetics & Toiletries 1990, 105, 53. One or more of the following aftertreatment components can be selected here: amino acids, beeswax, fatty acids, fatty acid alcohols, anionic surfactants, lecithin, phospholipids, sodium, potassium, zinc, iron or aluminium salts of fatty acids, polyethylenes, silicones, proteins (particularly collagen or elastin) , alkanolamines, silicon dioxide, aluminium oxide, further metal oxides, phosphates, such as sodium hexametaphosphate, or glycerine.

Particulate UV filters which are preferably employed here are:
- untreated titanium dioxides, such as, for example, the products Microtitanium Dioxide MT 500 B from Tayca; titanium dioxide P25 from Degussa;
- Aftertreated micronised titanium dioxides with aluminium oxide and silicon dioxide aftertreatment, such as, for example, the product "Microtitanium Dioxide MT 100 SA from Tayca, or the product "Tioveil Fin" from Uniqema;
- Aftertreated micronised titanium dioxides with aluminium oxide and/or aluminium stearate/laurate aftertreatment, such as, for example, Microtitanium Dioxide MT 100 T from Tayca; Eusolex T-2000 from Merck;
- Aftertreated micronised titanium dioxides with iron oxide and/or iron stearate aftertreatment, such as, for example, the product "Microtitanium Dioxide MT 100 F" from Tayca;
- Aftertreated micronised titanium dioxides with silicon dioxide, aluminium oxide and silicone aftertreatment, such as, for example, the product "Microtitanium Dioxide MT 100 SAS", from Tayca;
- Aftertreated micronised titanium dioxides with sodium hexametaphosphate, such as, for example, the product "Microtitanium Dioxide MT 150 W" from Tayca.

The treated micronised titanium dioxides employed for the combination may also be aftertreated with:
- Octyltrimethoxysilanes, such as, for example, the product Tego Sun T 805 from Degussa;
- Silicon dioxide; such as, for example, the product Parsol T-X from DSM;
- Aluminium oxide and stearic acid; such as, for example, the product UV-Titan M160 from Sachtleben;
- Aluminium and glycerine; such as, for example, the product UV-Titan from Sachtleben,
- Aluminium and silicone oils, such as, for example, the product UV-Titan M262 from Sachtleben;
- Sodium hexamethaphosphate and polyvinylpyrrolidone,
- Polydimethylsiloxanes, such as, for example, the product 70250 Cardre UF TiO2SI3" from Cardre;
- Polydimethylhydrogenosiloxanes, such as, for example, the product Microtitanium Dioxide USP Grade Hydrophobic" from Color Techniques.

The combination with the following products may furthermore also be advantageous:
- Untreated zinc oxides, such as, for example, the product Z-Cote from BASF (Sunsmart), Nanox from Elementis;
- Aftertreated zinc oxides, such as, for example, the following products:
   - "Zinc Oxide CS-5" from Toshibi (ZnO aftertreated with polymethyl-hydrogenosiloxane);
   - Nanogard Zinc Oxide FN from Nanophase Technologies;
   - "SPD-Z1" from Shin-Etsu (ZnO aftertreated with a silicone-grafted acrylic polymer, dispersed in cyclodimethylsiloxanes);
   - "Escalol Z100" from ISP (aluminium oxide-aftertreated ZnO, dispersed in an ethylhexyl methoxycinnamate/PVP-hexadecene/ methicone copolymer mixture);
   - "Fuji ZNO-SMS-10" from Fuji Pigment (ZnO aftertreated with silicon dioxide and polymethylsilesquioxane);
   - Untreated cerium oxide micropigment, for example with the name "Colloidal Cerium Oxide" from Rhone Poulenc;
   - Untreated and/or aftertreated iron oxides with the name Nanogar from Arnaud.

By way of example, it is also possible to employ mixtures of various metal oxides, such as, for example, titanium dioxide and cerium oxide, with and without aftertreatment, such as, for example, the product Sunveil A from Ikeda. In addition, mixtures of aluminium oxide-, silicon dioxide- and silicone-aftertreated titanium dioxide / zinc oxide mixtures, such as, for example, the product UV-Titan M261 from Sachtleben, can also be employed.

These inorganic UV filters are generally incorporated into the preparations in an amount of 0.1 to 25 per cent by weight, preferably 2 to 10% by weight.

By combination of one or more of the said compounds having a UV filter action, the protective action against harmful effects of the UV radiation can be optimised.

All said UV filters can also be employed in encapsulated form. In particular, it is advantageous to employ organic UV filters in encapsulated form. The capsules in preparations to be employed in accordance with the invention are preferably present in amounts which ensure that the encapsulated UV filters are present in the preparation in the per cent by weight ratios indicated above.

According to the present invention the formulation may preferably further comprise a pore-refining agent. Pore refining agents are, for example, retinol (vitamin A), 5,7-dihydroxy-2-methylchromone, marketed under the trade name RonaCare^{®} Luremine, nicotinamide or isoquercetin.

In a further preferred embodiment of the present invention the formulation further comprises at least one skin-lightening active compound (or synonymously depigmentation active compounds) or extracts having a skin-lightening activity.

Skin-lightening active compounds can in principle be all active compounds known to the person skilled in the art. Suitable for combination are commercially available melanogenesis inhibitors, such as, for example, ascorbic acid and derivatives thereof, aloesin, niacinamide, emblica, elagic acid, liquorice extract, mulberry extract, kojic acid, liquorice extract, rucinol, hydroquinone, azelaic acid, arbutin, magnesium ascorbyl phosphate, lactic acid, butylphenylmethoxyphenylpropandiol (available as RonaCare^{®} PristineBright^{®} from Merck) or the like. Preferred examples of compounds having skin-lightening activity are hydroquinone, niacinamide, ascorbic acid and physiologically acceptable salts thereof, kojic acid, arbutin, aloesin, azelaic acid, elagic acid, lactic acid, butylphenylmethoxyphenylpropandiol or rucinol. Preferred examples of extracts having skin-lightening activity are liquorice extract, mulberry extract or emblica.

The preparation as described above may further comprise one or more self-tanning substances. A preparation of this type generally has a contrast-reduction effect and enables a uniform skin shade to be achieved. The invention likewise relates to the use of compatible solutes, as described, in combination with one or more self-tanning substances for contrast reduction and achieving a uniform skin shade. A contrast-reduction agent is accordingly a substance which reduces a non-uniform skin coloration by reducing the contrast between more strongly and less strongly coloured skin areas. A uneven skin coloration of this type can arise here through non-uniform pigmentation and/or a different distribution of the horny skin. Uneven pigmentation is by no means unusual in the population and is based on different levels of melanin production by the melanocytes or an irregular distribution of the melanocytes in the skin.

A contrast reduction can be achieved, in particular, by preparations in which a self-tanning substance is further present. Such self-tanning substances may be self-tanning substances reacting with the amino acids of the skin based on a Maillard reaction or Michael addition, or may be so-called melanogenesis-prmoting substances or propigmentation substances, which promote the natural pigmentation of the skin.

Preferred self-tanning substances are, for example: 1,3-dihydroxyacetone (DHA) and derivatives derived therefrom, glycerolaldehyde, hydroxymethylglyoxal, γ-dialdehyde, erythrulose, 6-aldo-D-fructose, ninhydrin, 5-hydroxy-1,4-naphtoquinone (juglone) or 2-hydroxy-1,4-naphtoquinone (lawsone) or a mixture of the said compounds. Particularly preferred is 1,3-dihydroxyacetone, erythrulose and their mixture.

Propigmentation substances are known to the person skilled in the art. Examples are glycerrithinic acid, melanocyte-activating hormone (alpha-MSH), peptide analoga, thymidine-dinucleotide, L-tyrosine and their esters, bicyclic monoterpendiole (described in Brown et al., Photochemistry and Photobiology B: Biology 63 (2001) 148-161) or 7-acyloxy-chromen-4-on-derviatives (described in WO 2012/097857 A1), in particular hexadecanic acid 5-hydroxy-2-methyl-4-oxo-4H-chromen-7-yl ester (available from Merck KGaA, Darmstadt, Germany under Ronacare^{®} Bronzyl^{™}).

Perferably the self-tanning substance is present in the composition in an amount of 0,01 to 20 % by weight, more preferably in an amount of 0,5 to 15 % by weight and most preferably in an amount of 1 to 8 % by weight, related to the complete weight of the preparation.

In the preparations described coloured pigments may furthermore also be present, where the layer structure of the pigments is not limited. On use of 0.5 to 5% by weight, the coloured pigment should preferably be skin-coloured or brownish. The selection of a corresponding pigment is familiar to the person skilled in the art.

In a further preferred embodiment of the invention, the preparation comprises one or more antioxidants and/or one or more vitamins. The use of antioxidants enables a protective action against oxidative stress or against the effect of free radicals in general to be achieved, the person skilled in the art being presented with absolutely no difficulties in selecting antioxidants which act suitably quickly or with a time delay. There are many proven substances known from the specialist literature which can be used as antioxidants, for example amino acids (for example glycine, histidine, tyrosine, tryptophan) and derivatives thereof, imidazoles (for example urocanic acid) and derivatives thereof, peptides, such as, for example, D,L-carnosine, D-carnosine, L-carnosine and derivatives thereof (for example anserine), carotinoids, carotenes (such as, for example, α-carotene, β-carotene, lyco-pene) and derivatives thereof, chlorogenic acid and derivatives thereof, lipoic acid and derivatives thereof (such as, for example, dihydrolipoic acid), aurothioglucose, propylthiouracil and other thiols (such as, for example, thioredoxin, glutathione, cysteine, cystine, cystamine and the glycosyl, N-acetyl, methyl, ethyl, propyl, amyl, butyl and lauryl, palmitoyl, oleyl, γ-lino-leyl, cholesteryl and glyceryl esters thereof) and salts thereof, dilauryl thiodipropionate, distearyl thiodipropionate, thiodipropionic acid and derivatives thereof (such as, for example, esters, ethers, peptides, lipids, nucleotides, nucleosides and salts), and sulfoximine compounds (such as, for example, buthionine sulfoximines, homocysta sulfoximine, buthionine sulfones, penta-, hexa- and heptathionine sulfoximine) in very low tolerated doses (such as, for example, pmol to µmol/kg), and also (metal) chelating agents, (such as, for example, α-hydroxyfatty acids, palmitic acid, phytic acid, lactoferrin), α-hydroxy acids (such as, for example, citric acid, lactic acid, malic acid), humic acid, bile acid, bile extracts, bilirubin, biliverdin, EDTA, EGTA, pentasodium ethylenediamine tetramethylene phosphonate and derivatives thereof, unsaturated fatty acids and derivatives thereof, vitamin C and derivatives (such as, for example, ascorbyl palmitate, magnesium ascorbyl phosphate, ascorbyl acetate), tocopherols and derivatives (such as, for example, vitamin E acetate), vitamin A and derivatives (such as, for example, vitamin A palmitate) and coniferyl benzoate of benzoin resin, rutinic acid and derivatives thereof, α-glycosylrutin, ferulic acid, furfuryli-deneglucitol, carnosine, butylhydroxytoluene, butylhydroxyanisole, nor-dihydroguaiaretic acid, trihydroxybutyrophenone, quercetin, uric acid and derivatives thereof, mannose and derivatives thereof, zinc and derivatives thereof (such as, for example, ZnO, ZnSO₄), selenium and derivatives thereof (such as, for example, selenomethionine), stilbenes and derivatives thereof (such as, for example, stilbene oxide, trans-stilbene oxide). Further suitable antioxidants are also described in WO 2006/111233 and WO 2006/111234.

Suitable antioxidants are also compounds of the general formulae A or B 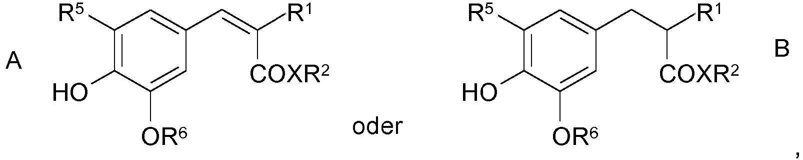 in which
- R¹: denotes -C(O)CH₃, -CO₂R³, -C(O)NH₂ and -C(O)N(R⁴)₂,
- X: denotes O or NH,
- R²: denotes linear or branched alkyl having 1 to 30 C atoms,
- R³: denotes linear or branched alkyl having 1 to 20 C atoms,
- R⁴: in each case, independently of one another, denotes H or linear or branched alkyl having 1 to 8 C atoms,
- R⁵: denotes H, linear or branched alkyl having 1 to 8 C atoms or linear or branched alkoxy having 1 to 8 C atoms, and
- R⁶: denotes linear or branched alkyl having 1 to 8 C atoms.

Preference is given to derivatives of 2-(4-hydroxy-3,5-dimethoxybenzylidene)malonic acid and/or 2-(4-hydroxy-3,5-dimethoxybenzyl)malonic acid, particularly preferably bis(2-ethylhexyl) 2-(4-hydroxy-3,5-dimethoxybenzylidene)malonate (for example Oxynex^{®} ST Liquid) and/or bis(2-ethylhexyl) 2-(4-hydroxy-3,5-dimethoxybenzy)malonate (for example RonaCare^{®} AP). Mixtures of antioxidants are likewise suitable for use in the preparations according to the invention. Known and commercial mixtures are, for example, mixtures comprising, as active ingredients, lecithin, L-(+)-ascorbyl palmitate and citric acid, natural tocopherols, L-(+)-ascorbyl palmitate, L-(+)-ascorbic acid and citric acid (such as, for example, Oxynex^{®} K LIQUID), tocopherol extracts from natural sources, L-(+)-ascorbyl palmitate, L-(+)-ascorbic acid and citric acid (such as, for example, Oxynex^{®} L LIQUID), DL-α-tocopherol, L-(+)-ascorbyl palmitate, citric acid and lecithin (such as, for example, Oxynex^{®} LM) or butylhydroxytoluene (BHT), L-(+)-ascorbyl palmitate and citric acid (such as, for example, Oxynex^{®} 2004). Antioxidants of this type are usually employed in such compositions with compounds as defined in claims in per cent by weight ratios in the range from 1000:1 to 1:1000, preferably in per cent by weight ratios of 100:1 to 1:100.

Of the phenols having an antioxidative action, the polyphenols, some of which are naturally occurring, are of particular interest for applications in the pharmaceutical, cosmetic or nutrition sector. For example, the flavonoids or bioflavonoids, which are principally known as plant dyes, frequently have an antioxidant potential. Lemanska et al., Current Topics in Biophysics 2000, 24(2), 101-108, are concerned with effects of the substitution pattern of mono- and dihydroxyflavones. It is observed therein that dihydroxyflavones containing an OH group adjacent to the keto function or OH groups in the 3'4'- or 6,7- or 7,8-position have antioxidative properties, while other mono-and dihydroxyflavones in some cases do not have antioxidative properties.

Quercetin (cyanidanol, cyanidenolon 1522, meletin, sophoretin, ericin, 3,3',4',5,7-pentahydroxyflavone) is frequently mentioned as a particularly effective antioxidant (for example Rice-Evans et al., Trends in Plant Science 1997, 2(4), 152-159). Lemanska et al., Free Radical Biology & Medicine 2001, 31(7), 869-881, have investigated the pH dependence of the antioxidant action of hydroxyflavones. Quercetin exhibits the highest activity amongst the structures investigated over the entire pH range.

The preparations according to the invention may comprise vitamins as further ingredients. Vitamins and vitamin derivatives selected from vitamin A, vitamin A propionate, vitamin A palmitate, vitamin A acetate, retinol, vitamin B, thiamine chloride hydrochloride (vitamin B₁), riboflavin (vitamin B₂), nicotinamide, vitamin C (ascorbic acid), vitamin D, ergocalciferol (vitamin D₂), vitamin E, DL-α-tocopherol, tocopherol E acetate, tocopherol hydro-gensuccinate, vitamin K1, esculin (vitamin P active compound), thiamine (vitamin B₁), nicotinic acid (niacin), pyridoxine, pyridoxal, pyridoxamine, (vitamin B₆), panthothenic acid, biotin, folic acid and cobalamine (vitamin B₁₂) are preferably present in the preparations according to the invention, particularly preferably vitamin A palmitate, vitamin C and derivatives thereof, DL-α-tocopherol, tocopherol E acetate, nicotinic acid, pantothenic acid and biotin. In the case of cosmetic application, vitamins are usually added with the preparations in ranges from 0.01 to 5% by weight, based on the total weight. Nutrition-physiological applications are oriented towards the respective recommended vitamin requirement.

The formulation according to the present invention may further comprise at least one substance which serves for maintaining and/or improving the moisture content of the skin. These substances can, without this being intended to be regarded as a restriction, also be, inter alia, substances which belong to the so-called natural moisturising factors, such as, for example, 2-oxopyrrolidine 5-carboxylic acid.

The preparations according to the invention may in addition comprise anti-ageing active compounds, anticellulite active compounds or conventional skin-protecting or skin-care active compounds. Skin-protecting or skin-care active compounds can in principle be all active compounds known to the person skilled in the art. Particularly preferred anti-ageing active compounds are pyrimidinecarboxylic acids, aryl oximes, bioflavonoids, bio-flavonoid-containing extracts, chromones or retinoids.

Additionally, anti-ageing active compounds which can be used are products from Merck, such as, for example, 5,7-dihydroxy-2-methylchromone, marketed under the trade name RonaCare^{®} Luremine, Ronacare^{®} Isoquercetin, Ronacare^{®} Tilirosid or Ronacare^{®} Cyclopeptide 5.

Known anti-ageing substances are also chromones, as described, for example, in EP 1508327, or retinoids, for example retinol (vitamin A), reti-noic acid, retinaldehyde or also synthetically modified compounds of vitamin A. The chromones and retinoids described are simultaneously also effective anticellulite active compounds. An anticellulite active compound which is likewise known is caffeine.

In the above-mentioned formulations, the compatible solutes can advantageously be combined with all known preservatives or antimicrobial active compounds, such as, for example, anisic acid, alcohol, ammomium benzoate, ammonium propionate, benzoic acid, bronopol, butylparaben, benzethonium chloride, benzalkonium chloride, 5-bromo-5-nitro-1,3-dioxane, benzyl alcohol, boric acid, benzisothiazolinone, benzotriazole, benzyl hemiformate, benzylparaben, 2-bromo-2-nitropropane-1,3-diol, butyl benzoate, chlorphenesin, capryl/capric glycerides, caprylyl glycol, Camellia Sinensis leaf extract, Candida Bombicola/glucose/methyl rapeseedates, chloroxylenol, chloroacetamide, chlorhexidine, chlorobutanol, calcium benzoate, calcium paraben, calcium propionate, calcium salicylate, calcium sorbate, captan, chloramine T, chlorhexidine diacetate, chlorhexidine digluconate, chlorhexidine dithydrochloride, chloroacetamine, p-chloro-m-cresol, chlorphen, p-chlorophenol, chlorothymol, Citrus Grandis (grapefruit) fruit extract, Citrus Grandis (grapefruit) seed extract, m-cresol, o-cresol, p-cresol, mixed cresols, 1,2-decanediol (INCI Decylene Glycol), diazolidinylurea, dichlorobenzyl alcohol, dimethyloxazolidine, DMDM hydantoin, dimethylhydroxmethylpyrazole, dehydroacetic acid, diazolidinylurea, DEDM hydantoin, DEDM hydantoin dilaurate, dibromopropamidine diisothionate, dimethylolethylenethiourea, dithio-methylbenzamide, DMHF, domiphen bromide, 7-ethylbicyclooxazolidine, ethylparaben, ethylhexylglycerol, ethanol, ethyl ferulate, formaldehyde, ferulic acid, glyceryl caprate, glutaral, glycerol formate, glyoxal, hexamidine diisethionate, hexanediol, hexetidine, hexamidine, hexamidinediparaben, hexamidineparaben, 4-hydroxybenzoic acid, hydroxymethyldioxazabicyclo-octane, imidazolidinylurea, imidiazolidinylurea NF, isobutylparaben, isothia-zolinone, iodopropynylbutyl carbamate, isodecylparaben, isopropylcresol, isopropylparaben, isopropyl sorbate, potassium sorbate NF FCC, copper usnate, potassium benzoate, potassium ethylparaben, potassium methylparaben, potassium paraben, potassium phenoxide, potassium o-phenylphenate, potassium propionate, potassium propylparaben, potassium salicylate, potassium sorbate, methylparaben, methylisothiazolinone, methylbenzethonium chloride phenol, methyldibromoglutaronitrile, methen-ammonium chloride, methylbromoglutaronitrile, magnesium benzoate, magnesium propionate, magnesium salicylate, MDM hydantoin, MEA benzoate, MEA o-phenylphenate, MEA salicylate, methylchloristhiazolin-one, sodium benzoate NF FCC, sodium caprylate, sodium dehydroacetate, sodium dehydroacetates FCC, sodium hydroxymethylglycinate, sodium methylparaben, sodium propylparaben, sodium iodoate, neem tree seed oil, nisin, sodium benzoate, sodium butylparaben, sodium p-chloro-m-cresol, sodium ethylparaben, sodium formate, sodium hydroxymethanesulfonate, sodium isobutylparaben, sodium paraben, sodium phenol sulfonate, sodium phenoxide, sodium o-phenylphenate, sodium propionate, sodium propylparaben, sodium pyrithione, sodium salicylate, sodium sorbate, orthol-phenylphenol, phenoxyethanol, propylparaben, polymethoxybicyclicoxa-zolidine, Pinus Pinaster bark extract, poloxamer 188, PVP iodine, para-bens, pircotone olamines, phenethyl alcohol, polyaminopropylbiguanide, polyquarternium-42, PEG-5 DEDM hydantoin, PEG-15 DEDM hydantoin, PEG-5 hydantoin oleate, PEG-15 DEDM hydantoin stearate, phenethyl alcohol, phenol, phenoxyethylparaben, phenoxyisopropanol, phenyl benzoate, phenyl mercury acetate, phenyl mercury benzoate, phenyl mercury borate, phenyl mercury bromide, phenyl mercury chloride, phenylparaben, o-phenylphenol, polyaminopropylbiguanide stearate, propionic acid, propyl benzoate, quaternium-15, quaternium-8, quaternium-14, Rosmarinus offici-nalis leaf extract, sorbic acid NF FCC, selenium disulfine, sorbic acid, sali-cylic acid, silver borosilicate, silver magnesium aluminium phosphate, triclosan, di-alpha-tocopherol, tocopherol acetate, thimersal, triclocarban, TEA sorbate, thimerosal, usnic acid, undecylenoyl PEG-5 paraben, Vitis vinifera seed extract, tea tree oil, hydrogen peroxide, zinc pyrithione, zinc oxide, zinc phenolsulfonate or combinations thereof.

Within this regard, the formulation may comprise the antimicrobially active compounds described in WO 2013/091775 A2, WO 2013/159865 A1 or WO 2013/167220 A1, in particular 4-hydroxy-cyclohexanecarboxylic acid butyl ester (available as RonaCare^{®} SereneShield from Merck KGaA, Darmstadt, Germany).

The formulation may further comprise anti-acne active compounds, for example, in WO2009/098139 on page 47, line 2 to page 48, line 27 and DE10324567, are conceivable. Illustrative further anti-acne active compounds are silver particles and silver salts, such as silver lactate and silver citrate, azelaic acid, ellagic acid, lactic acid, glycolic acid, salicylic acid, glycyrrhizinic acid, triclosan, phenoxyethanol, hexamidine isethionate, ketoconazole, peroxides, such as hydrogen peroxide or benzoyl peroxide, 3-hydroxybenzoic acid, 4-hydroxybenzoic acid, phytic acid, arachidonic acid, caprylyl glycol, ethylhexylglycerol, farnesol, cetylpyridinium salts, 6-trimethylpentyl-2-pyridone (Piroctone Olamine) and lipohydroxy acid (LHA).

Antidandruff active compounds are, for example, zinc pyrithione, Piroctone Olamine, selenium disulfide, Climbazole, Triclosan, Butylparaben, 1,3-Bis(hydroxymethyl)-5,5-dimethylimidazolidine-2,4-dione (DMDM Hydantoin), fumaric acid, Methylchloroisothiazolinone or Methylisothiazolinone (MIT).

The following, for example, may be mentioned as use form of the preparations according to the invention: solutions, suspensions, emulsions, PIT emulsions, pastes, ointments, gels, creams, lotions, foams, masks, powders, soaps, surfactant-containing cleansing preparations, oils, aerosols, plasters, compresses, bandages and sprays, in particular for external application. Further application forms are, for example, sticks, shampoos and shower baths. Typcal cosmetic use forms are furthermore also lipsticks, lip-care sticks, powder, emulsion and wax make-up, and sun-protection, pre-sun and after-sun preparations.

Cosmetic and dermatological preparations according to the invention can be, in particular, a water-free preparation, a lotion or emulsion, such as cream or milk, or microemulsion, in each case of the water-in-oil (W/O) type or of the oil-in-water (O/W) type, a multiple emulsion, for example of the water-in-oil-in-water (W/O/W) type or vice versa (O/W/O), gels or solutions (in particular oily-alcoholic, oily-aqueous or aqueous-alcoholic gels or solutions), a solid stick, an ointment or an aerosol. For application, the cosmetic and dermatological preparations according to the invention are applied to the skin in adequate amount in the usual manner for cosmetics.

Any desired conventional vehicles, assistants and, if desired, further active compounds may be added to the preparation. Preferred assistants originate from the group of the preservatives, stabilisers, solubilisers, colorants, i.e. pigments, dyes, emulsifiers or odour improvers.

Ointments, pastes, creams and gels may comprise the customary vehicles which are suitable for topical application, such as, for example, animal and vegetable fats, waxes, paraffins, starch, tragacanth, cellulose derivatives, polyethylene glycols, silicones, bentonites, silica, talc and titanium dioxide, or mixtures of these substances.

Powders and sprays may comprise the customary vehicles, such as, for example, lactose, talc, silica, aluminium hydroxide, calcium silicate and polyamide powder, or mixtures of these substances. Sprays may additionally comprise the customary readily volatile, liquefied propellants, such as, for example, chlorofluorocarbons, propane/butane or dimethyl ether. Compressed air can also advantageously be used. However, air can also be employed in pressureless metering devices, such as, for example, pump sprays.

Solutions and emulsions may comprise the customary vehicles, such as solvents, solubilisers and emulsifiers, such as, for example, water, ethanol, isopropanol, ethyl carbonate, ethyl acetate, benzyl alcohol, benzyl benzoate, propylene glycol, 1,3-butyl glycol, oils, in particular cottonseed oil, peanut oil, wheatgerm oil, olive oil, castor oil and sesame oil, glycerol fatty acid esters, polyethylene glycols and fatty acid esters of sorbitan, or mixtures of these substances.

A preferred solubiliser in general is 2-isopropyl-5-methylcyclohexane-carbonyl-D-alanine methyl ester.

Suspensions may comprise the customary vehicles, such as liquid diluents, such as, for example, water, ethanol or propylene glycol, suspension media, such as, for example, ethoxylated isostearyl alcohols, polyoxyethylene sorbitol esters and polyoxyethylene sorbitan esters, microcrystalline cellulose, aluminium metahydroxide, bentonite, agar-agar and tragacanth, or mixtures of these substances.

Soaps may comprise the customary vehicles, such as alkali-metal salts of fatty acids, salts of fatty acid monoesters, fatty acid protein hydrolysates, isothionates, lanolin, fatty alcohol, vegetable oils, plant extracts, glycerol, sugars, or mixtures of these substances.

Surfactant-containing cleansing products may comprise the customary vehicles, such as salts of fatty alcohol sulfates, fatty alcohol ether sulfates, sulfosuccinic acid monoesters, fatty acid protein hydrolysates, isothionates, imidazolinium derivatives, methyl taurates, sarcosinates, fatty acid amide ether sulfates, alkylamidobetaines, fatty alcohols, fatty acid glycerides, fatty acid diethanolamides, vegetable and synthetic oils, lanolin derivatives, ethoxylated glycerol fatty acid esters, or mixtures of these substances.

Face and body oils may comprise the customary vehicles, such as synthetic oils, such as fatty acid esters, fatty alcohols, silicone oils, natural oils, such as vegetable oils and oily plant extracts, paraffin oils, lanolin oils, or mixtures of these substances.

Further suitable vehicles are liposomes, cyclodextrines or other sugars such as sorbitol.

A preferred embodiment of the invention is an emulsion which is in the form of a cream or milk and comprises, for example, the said fats, oils, waxes and other fatty substances, as well as water or an aqueous phase, for example with solvents or hydrophilic surfactants, and an emulsifier, as usually used for a preparation of this type.

The lipid phase can advantageously be selected from the following substance group:
- mineral oils, mineral waxes;
- oils, such as, for example, triglycerides of capric or caprylic acid, furthermore natural oils, such as, for example, castor oil;
- fats, waxes and other natural and synthetic fatty substances, preferably esters of fatty acids with alcohols having a low carbon number, for example with isopropanol, propylene glycol or glycerol, or esters of fatty alcohols with alkanoic acids having a low carbon number or with fatty acids;
- silicone oils, such as dimethylpolysiloxanes, diethylpolysiloxanes, diphenylpolysiloxanes, and mixed forms thereof.

For the purposes of the present invention, the oil phase of the emulsions, oleogels or hydrodispersions or lipodispersions is advantageously selected from the group of esters of saturated and/or unsaturated, branched and/or unbranched alkanecarboxylic acids having a chain length of 3 to 30 C atoms and saturated and/or unsaturated, branched and/or unbranched alcohols having a chain length of 3 to 30 C atoms, from the group of the esters of aromatic carboxylic acid and saturated and/or unsaturated, branched and/or unbranched alcohols having a chain length of 3 to 30 C atoms. Ester oils of this type can then advantageously be selected from the group isopropyl myristate, isopropyl palmitate, isopropyl stearate, isopropyl oleate, n butyl stearate, n-hexyl laurate, n-decyl oleate, isooctyl stearate, isononyl stearate, isononyl isononanoate, 2-ethylhexyl palmitate, 2-ethylhexyl laurate, 2-hexaldecyl stearate, 2-octyldodecyl palmitate, oleyl oleate, oleyl erucate, erucyl oleate, erucyl erucate and synthetic, semi-synthetic and natural mixtures of esters of this type, such as, for example, jojoba oil.

Furthermore, the oil phase can advantageously be selected from the group of branched and unbranched hydrocarbons and hydrocarbon waxes, silicone oils, dialkyl ethers, the group of saturated or unsaturated, branched or unbranched alcohols, and fatty acid triglycerides, specifically the triglycerol esters of saturated and/or unsaturated, branched and/or unbranched alkanecarboxylic acids having a chain length of 8 to 24, in particular 12-18 C atoms. The fatty acid triglycerides can advantageously be selected, for example, from the group of synthetic, semi-synthetic and natural oils, for example olive oil, sunflower oil, soya oil, peanut oil, rapeseed oil, almond oil, palm oil, coconut oil, palm kernel oil and the like.

Any desired mixtures of oil and wax components of this type may also advantageously be employed for the purposes of the present invention. It may also be advantageous to employ waxes, for example cetyl palmitate, as sole lipid component of the oil phase.

The aqueous phase of the preparations according to the invention optionally advantageously comprises alcohols, diols or polyols having a low carbon number, and ethers thereof, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products, furthermore alcohols having a low carbon number, such as, for example, ethanol, isopropanol, 1,2-propanediol, glycerol, and, in particular, one or more thickeners, which may advantageously be selected from the group of silicon dioxide, aluminium silicates, polysaccharides or derivatives thereof, such as, for example, hyaluronic acid, xanthan gum, hydroxypropylmethylcellulose, particularly advantageously from the group of the polyacrylates, preferably a polyacrylate from the group of the so-called Carbopols, for example Carbopol grades 980, 981, 1382, 2984, 5984, in each case individually or in combination. In particular, mixtures of the above-mentioned solvents are used. In the case of alcoholic solvents, water may be a further constituent.

In a preferred embodiment, the preparations according to the invention comprise hydrophilic surfactants. The hydrophilic surfactants are preferably selected from the group of the alkylglucosides, acyl lactylates, betaines and coconut amphoacetates.

Emulsifiers that can be used are, for example, the known W/O and O/W emulsifiers. It is advantageous to use further conventional co-emulsifiers in the preferred O/W emulsions according to the invention.

The co-emulsifiers selected in accordance with the invention are advantageously, for example, O/W emulsifiers, principally from the group of substances having HLB values of 11-16, very particularly advantageously having HLB values of 14.5-15.5, so long as the O/W emulsifiers have saturated radicals R and R'. If the O/W emulsifiers have unsaturated radicals R and/or R', or if isoalkyl derivatives are present, the preferred HLB value of such emulsifiers may also be lower or higher.

It is advantageous to select the fatty alcohol ethoxylates from the group of the ethoxylated stearyl alcohols, cetyl alcohols, cetylstearyl alcohols (cetearyl alcohols).

It is furthermore advantageous to select the fatty acid ethoxylates from the following group:
polyethylene glycol (20) stearate, polyethylene glycol (21) stearate, polyethylene glycol (22) stearate, polyethylene glycol (23) stearate, polyethylene glycol (24) stearate, polyethylene glycol (25) stearate, polyethylene glycol (12) isostearate, polyethylene glycol (13) isostearate, polyethylene glycol (14) isostearate, polyethylene glycol (15) isostearate, polyethylene glycol (16) isostearate, polyethylene glycol (17) isostearate, polyethylene glycol (18) isostearate, polyethylene glycol (19) isostearate, polyethylene glycol (20) isostearate, polyethylene glycol (21) isostearate, polyethylene glycol (22) isostearate, polyethylene glycol (23) isostearate, polyethylene glycol (24) isostearate, polyethylene glycol (25) isostearate, polyethylene glycol (12) oleate, polyethylene glycol (13) oleate, polyethylene glycol (14) oleate, polyethylene glycol (15) oleate, polyethylene glycol (16) oleate, polyethylene glycol (17) oleate, polyethylene glycol (18) oleate, polyethylene glycol (19) oleate, polyethylene glycol (20) oleate.

An ethoxylated alkyl ether carboxylic acid or salt thereof which can advantageously be used is sodium laureth-11 carboxylate. An alkyl ether sulfate which can advantageously be used is sodium laureth1-4 sulfate. An ethoxylated cholesterol derivative which can advantageously be used is polyethylene glycol (30) cholesteryl ether. Polyethylene glycol (25) soya-sterol has also proven successful. Ethoxylated triglycerides which can advantageously be used are the polyethylene glycol (60) evening primrose glycerides.

It is furthermore advantageous to select the polyethylene glycol glycerol fatty acid esters from the group of polyethylene glycol (20) glyceryl laurate, polyethylene glycol (21) glyceryl laurate, polyethylene glycol (22) glyceryl laurate, polyethylene glycol (23) glyceryl laurate, polyethylene glycol (6) glyceryl caprate/cprinate, polyethylene glycol (20) glyceryl oleate, polyethylene glycol (20) glyceryl isostearate, polyethylene glycol (18) glyceryl oleate (cocoate).

It is likewise favourable to select the sorbitan esters from the group polyethylene glycol (20) sorbitan monolaurate, polyethylene glycol (20) sorbitan monostearate, polyethylene glycol (20) sorbitan monoisostearate, polyethylene glycol (20) sorbitan monopalmitate and polyethylene glycol (20) sorbitan monooleate.

The following can be employed as optional W/O emulsifiers, but ones which may nevertheless be advantageous in accordance with the invention: fatty alcohols having 8 to 30 carbon atoms, monoglycerol esters of saturated and/or unsaturated, branched and/or unbranched alkanecarboxylic acids having a chain length of 8 to 24, in particular 12 to 18 C atoms, diglycerol esters of saturated and/or unsaturated, branched and/or unbranched alkanecarboxylic acids having a chain length of 8 to 24, in particular 12 to 18 C atoms, monoglycerol ethers of saturated and/or unsaturated, branched and/or unbranched alcohols having a chain length of 8 to 24, in particular 12 to 18 C atoms, diglycerol ethers of saturated and/or unsaturated, branched and/or unbranched alcohols having a chain length of 8 to 24, in particular 12 to 18 C atoms, propylene glycol esters of saturated and/or unsaturated, branched and/or unbranched alkanecarboxylic acids having a chain length of 8 to 24, in particular 12 to 18 C atoms, and sorbitan esters of saturated and/or unsaturated, branched and/or unbranched alkanecarboxylic acids having a chain length of 8 to 24, in particular 12 to 18 C atoms.

Particularly advantageous W/O emulsifiers are glyceryl monostearate, glyceryl monoisostearate, glyceryl monomyristate, glyceryl monooleate, diglyceryl monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol, polyethylene glycol (2) stearyl ether (steareth-2), glyceryl monolaurate, glyceryl monocaprinate, glyceryl monocaprylate or PEG-30 dipolyhydroxystearate.

The preparation may comprise cosmetic adjuvants which are usually used in this type of preparation, such as, for example, thickeners, softeners, moisturisers, surface-active agents, emulsifiers, preservatives, antifoams, perfumes, waxes, lanolin, propellants, dyes and/or pigments, which colour the composition itself or the skin, and other ingredients usually used in cosmetics.

The dispersant or solubiliser used can be an oil, wax or other fatty substances, a lower monoalcohol or a lower polyol or mixtures thereof. Particularly preferred monoalcohols or polyols include ethanol, i-propanol, propylene glycol, glycerol and sorbitol.

Further preferred embodiments are oily lotions based on natural or synthetic oils and waxes, lanolin, fatty acid esters, in particular triglycerides of fatty acids, or oily-alcoholic lotions based on a lower alcohol, such as ethanol, or a glycerol, such as propylene glycol, and/or a polyol, such as glycerol, and oils, waxes and fatty acid esters, such as triglycerides of fatty acids.

The preparation according to the invention may also be in the form of an alcoholic gel which comprises one or more lower alcohols or polyols, such as ethanol, propylene glycol or glycerol, and a thickener, such as siliceous earth. The oily-alcoholic gels additionally comprise natural or synthetic oil or wax.

The solid sticks preferably consist of natural or synthetic waxes and oils, fatty alcohols, fatty acids, fatty acid esters, lanolin and other fatty substances.

If a preparation is formulated as an aerosol, the usual propellants, such as alkanes, air, nitrogen, dinitrogen monoxide, particularly preferably alkanes or air, are preferably used.

A further embodiment of the present invention is a formulation as described above, characterized in that a vehicle which is suitable for topical applications and optionally physiologically acceptable assistants and/or fillers are present. Such vehicle, assistants and fillers are defined as described above.

Another embodiment of the present invention is directed to a process for the preparation of a preparation as described above, characterized in that the at least one compatible solute is mixed with the at least one further skin pore lightening active and optionally with the further ingredients.

These compounds can be incorporated into cosmetic or dermatological preparations in the usual manner.

The process for the preparation typically comprises the following steps:
(a) mixing of at least one compatible solute with at least one further pore-lightening active compound and at least one vehicle which is suitable for topical applications and optionally with physiologically acceptable assistants and/or fillers, and optionally (b) apparent making-ready of the compatible solutes. The preparations according to the invention can be prepared with the aid of techniques which are well known to the person skilled in the art. corneum.

The compositions as described above are applied to the skin.

Even without further comments, it is assumed that a person skilled in the art will be able to utilise the above description in its broadest scope. The preferred embodiments should therefore merely be regarded as descriptive disclosure which is absolutely not limiting in any way.

The following examples are intended to illustrate the present invention. However, they should in no way be regarded as limiting.

### Examples

### Example 1: Development of cells with intracellularly accumulated carbonylated proteins

Protein carbonylation is initiated by reactive aldehyde compounds which are synthesized by lipid peroxidation. Since skin pores are always immersed by sebum, oleic acid which is one of the sebum components, is used as a source of intracellular synthesis of reactive aldehyde compounds to establish cells with accumulated carbonylated proteins.

### Methods:

Cell culture: HaCaT keratinocytes are cultured in Dulbeco's modified Eagle's medium (DMEM) (Nissui, Tokyo, Japan) supplemented with 5% fetal bovine serum (FBS) (Thermofisher, Waltham, CA, USA) at 37°C in 5% CO₂ and 95% air.

For this example HaCaT keratinocytes are cultured in DMEM containing oleic acid at several concentrations for 48 h. After fixing with cold methanol for 5 min, cells are reacted with 20 µmol/L FTSC (Sigma-Aldrich, St. Louis, MO, USA in 0.1 mol/L MES-Na buffer (pH 5.5) at room temperature for 1 h. Fluorescence intensities derived from CPs are measured at Ex/Em 492nm /516nm with a fluorescence microplate reader, TECAN SPARK 10M (Männedorf,Switzerland). Numbers of cells are determined by measuring fluorescence intensities of nuclei stained with Hoechst 33342 (Sigma-Aldrich (St. Louis, MO, USA)). Intracellular CP levels are calculated as fluorescence intensity of FTSC per fluorescence intensities of nuclei.

### Results:

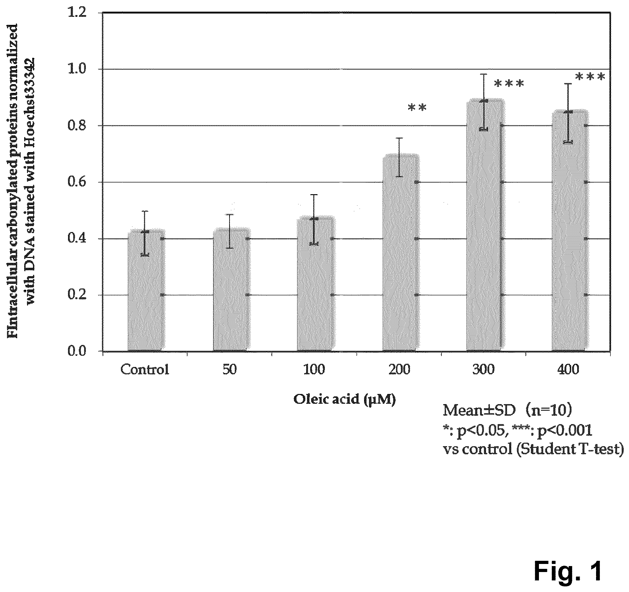

The results are shown in Figure 1: HaCaT keratinocytes cultured with oleic acid show a higher level of intracellular CPs

### Example 2: Effect of ectoin on suppression of intracellular carbonylated protein accumulation

The effect of ectoin on carbonylated protein accumulation is analysed.

In order to induce Hsp70, HaCaT keratinocytes are cultured with 100mM ectoin (Merck KGaA, Germany) for 24 h. Cells are cultured with oleic acid for further 48 hours, and CP levels are determined. In this examination, 200 µM oleic acid is used to evaluate the effect of ectoin on CP accumulation in the cells.

### Methods:

HaCaT keratinocytes are cultured in DMEM containing 5 % FBS and 100 mM ectoin for 24 h. Cells are further cultured with DMEM containing 200 µM oleic acid for 48 h. After fixing with cold methanol for 5 min, cells are reacted with 20 µmol/L FTSC in 0.1 mol/L MES-Na buffer (pH 5.5) at room temperature for 1 h. Fluorescence intensities derived from CPs are measured at Ex/Em 492nm /516nm with a fluorescence microplate reader, TECAN SPARK 10M (Männedorf, Switzerland). Numbers of cells are determined by measuring fluorescence intensities of nuclei stained with Hoechst 33342. Intracellular CP levels are calculated as fluorescence intensity of FTSC per fluorescence intensities of nuclei.

### Results:

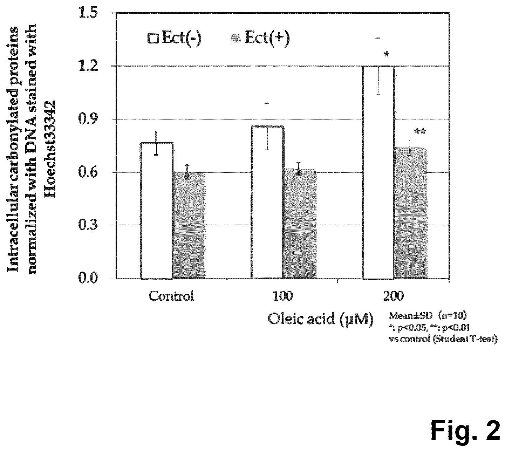

Cells pre-treated with ectoin show a suppression of CP accumulation through oleic acid (Figure 2).

### Example 3: Protective effect of ectoin against UVA and blue light induced carbonylated protein formation

The protective activity of ectoin against UVA and blue light induced carbonylated protein formation is assessed.

### Methods:

HaCaT keratinocytes are cultured in calcium-free DMEM (Dulbecco's Modified Eagle Medium), with 10% SVF, at 37 °C and humid atmosphere, supplemented with 5% CO2 for 24h.

After this initial time period the cell cultures are treated with the respective active compound. A non treated sample is used as reference.

The following active compounds are used in the above method:
- Ectoin (Merck KGaA, Germany) 0.5% w/v dissolved in culture medium.
- Ectoin (Merck KGaA, Germany) 0.3% w/v dissolved in culture medium
- N-acetyl-cysteine (NAC) (Sigma-Aldrich) 2,5 mM, as internal reference

After a time period of 24 h, stress is induced in the samples by treatment with blue light irradiation (460 nm) or UVA irradiation (365 nm). In addition, samples not treated with an active compound are left non-irradiated for comparison.

Blue light irradiation (460 nm):
- Source: LED
- Irradiance: 53 mW/cm²
- Dose: 35 J/cm² (corresponding to the emission of a computer screen during 8h/d for 3 days)
- Irradiation time: 11 min

UVA irradiation (365 nm):
- Source: LED
- Irradiance: 50 mW/cm²
- Dose: 33 J/cm² (i.e. less intense than 15 minutes at noon in the middle European sunlight)
- Irradiation time: 11 min

After stress induction the samples are immediately quick frozen at -80°C in liquid nitrogen for the analysis.

Three samples are prepared for each tested condition.

In situ detection of carbonylated proteins: After thawing of the samples, protein extraction from cells is performed using OxiProteomics' validated protocols. Extracted proteins are solubilized and quantified by the Bradford method (Bradford, 1976, Anal. Biochem., 72, 248) using calibrated BSA as standard. The samples are split into 3 equal amounts for the analyses. Carbonylated proteins are labeled with specific functionalized fluorescent probes (using Cy3 NHS (-hydroxysulfosuccinimide)) routinely used in proteomics. Proteins are then resolved by high-resolution electrophoresis separation (4-20 % gradient SDS-PAGE; buffer trys-glycine (Biorad)). Proteins are fixed to the gel. Total proteins are post-stained with SyproRuby TM protein gel stain. Image acquisition for carbonylated and total proteins is performed using the Ettan^{®} DIGE imager (GE Healthcare). Densitomertric analysis of protein bands is performed using ImageJ analysis software (Rasband, W.S., ImageJ, U.S. National Institute of Health, Bethesda, Maryland, USA, http://imagej.nih.gov/ij/, 1997-B014). Quantification is obtained from each sample, both for carbonylated and total proteins.

### Results:

The results are shown in figures 3 to 6:
Figures 3 and 5 show the images acquired after SDS-PAGE for samples with UVA irradiation (figure 3) and samples with blue light irradiation (figure 5). The left gels in figure 3 and 5 show the fluorescent labelled carbonylated proteins; the right gels in figure 3 and 5 show the total post-stained proteins.

Figures 4 (UVA irradiation) and 6 (blue light irradiation) show the quantitative analysis of the SDS-PAGE gels according to figure 3 and 5, respectively.

### Sample codes:

CTRL = non-treated, non-irradiated control
UVA = non-treated, irradiated with UVA
BL/ Blue Light = non-treated, irradiated with blue light
NAC = N-acetyl-cystein treated, irradiated with either UVA or blue light
Ectoin 0.3 = treated with ectoine 0.3% w/v, irradiated with either UVA or blue light
Ectoin 0.5 = treated with ectoine 0.5% w/v, irradiated with either UVA or blue light

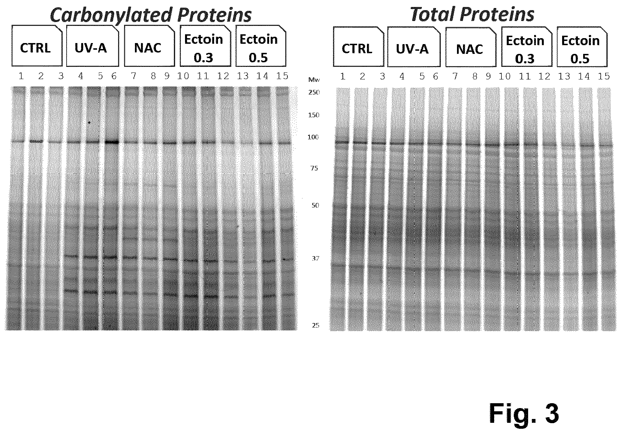

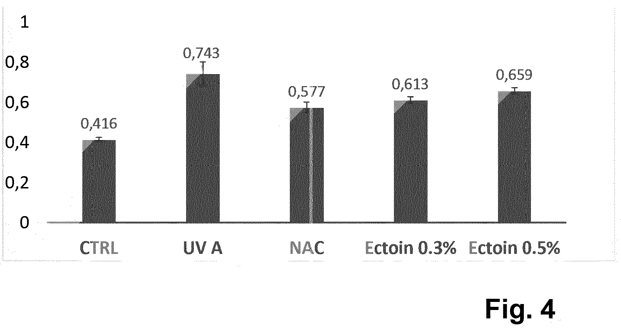

Figures 3 and 4 show that ectoin has a significant protective effect against UVA induced protein carbonylation at a concentration of 0.3% in comparison to the control. No significant comparison between the control and ectoin at a concentration of 0.5% is observed due to the fact that the statistical deviation of measured data is too high to enable a solid interpretation.

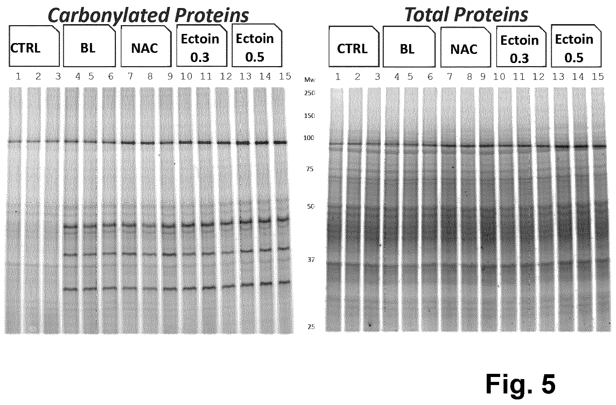

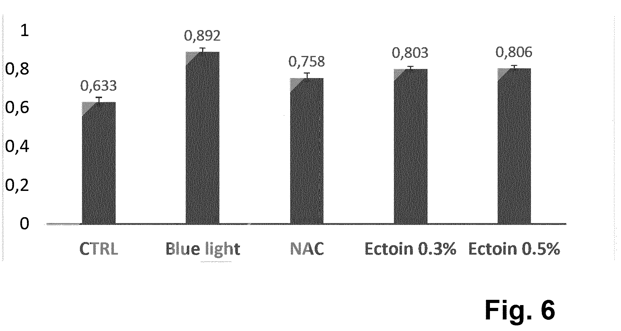

Figures 5 and 6 show that ectoine has a significant protective effect against blue light induced protein carbonylation at a concentration of 0.3% and 0.5%. The performance is comparable for both use levels.

### Example 4: Aqueous mask for sensitive and dehydrated skin

| **Ingredients** | | **INCI (EU)** | **[%]** |
|---|---|---|---|
| **A** | | | |
| RonaCare^{®} Biotin Plus | (1) | UREA, DISODIUM PHOSPHATE, BIOTIN, CITRIC ACID | 1.00 |
| RonaCare^{®} Luremin^{®} | (1) | SORBITOL, DIHYDROXY METHYLCHROMONE | 2.00 |
| RonaCare^{®} VTA | (1) | AQUA, ALCOHOL, LECITHIN, DISODIUM RUTINYL DISULFATE, HYDROXYPROLINE, SORBITOL, MAGNESIUM ASCORBYL PHOSPHATE, TOCOPHEROL, ASCORBYL PALMITATE, GLYCERYL STEARATE, GLYCERYL OLEATE, CITRIC ACID | 4.00 |
| RonaCare^{®} Ectoin | (1) | ECTOIN | 2.00 |
| Glycerol 85% | (1) | GLYCERIN, AQUA | 8.00 |
| COVACRYL MV 60 | (2) | SODIUM POLYACRYLATE | 3.00 |
| Water, demineralized | | AQUA | ad 100 |

| **B** | | | |
|---|---|---|---|
| RonaFlair^{®} Boroneige^{®} SF-3 | (1) | BORON NITRIDE | 1.00 |

| **C** | | | |
|---|---|---|---|
| Preservatives | | | q.s. |

### Procedure:

Mix the water, the glycerin and the active ingredients of phase A. Stir until homogeneous. Sprinkle Covacryl MV60 under low stirring and then increase the stirring and stir until homogeneous. Add B and stir until homogeneous. Add C and stir until homogeneous. Adjust to pH 6,5 if necessary.
Notes: (1cP is 1mPa.s)
Appearance : white gel
pH : 6.5
Viscosity : 40 000 cP (Fungilab Expert, Spindle PB, 5 rpm)
Stability : 3 months at RT, 4°C and 45°C
Suppliers:
   (1) Merck KGaA, Darmstadt, Germany / EMD Performance Materials
   (2) Sensient Cosmetic Technologies

### Example 5: Soothing face mask for sensitive and dry skin

| **Ingredients** | | **INCI (EU)** | **[%]** |
|---|---|---|---|
| **A** | | | |
| RonaCare^{®} Ectoin | (1) | ECTOIN | 1.00 |
| RonaCare^{®} Nicotinamide | (1) | NIACINAMIDE | 2.00 |
| RonaCare^{®} Disodium | (1) | DISODIUM EDTA | 0.05 |
| EDTA | | | |
| Carbopol^{®} Ultrez 21 | (2) | ACRYLATES/C10-30 ALKYL | 0.20 |
| | | ACRYLATE CROSSPOLYMER | |
| Glycerol 85% | (1) | GLYCERIN, AQUA | 5.00 |
| Rhodicare XC | (3) | XANTHAN GUM | 0.30 |
| Water, demineralized | | AQUA | ad 100 |
| Montanov L | (4) | C14-22 ALCOHOLS, C12-20 ALKYL GLUCOSIDE | 4.00 |

| | | | |
|---|---|---|---|
| Montanov 14 | (4) | MYRISTYL ALCOHOL, MYRISTYL GLUCOSIDE | 2.00 |

| **B** | | | |
|---|---|---|---|
| RonaCare^{®} AP | (1) | BIS-ETHYLHEXYL | 1.00 |
| | | HYDROXYDIMETHOXY | |
| | | BENZYLMALONATE | |
| RonaCare^{®} Bisabolol | (1) | BISABOLOL | 0.50 |
| Cetyl Alcohol | (1) | CETYL ALCOHOL | 1.00 |
| LIPOCIRE A PELLETS | (2) | C10-18 TRIGLYCERIDES | 3.00 |
| Cetiol SB 45 | (5) | BUTYROSPERMUM PARKII BUTTER | 5.00 |
| Mango butter XNE003 | (6) | MANGIFERA INDICA SEED BUTTER | 5.00 |
| Crodamol ISIS-LQ-(MV) | (7) | ISOSTEARYL ISOSTEARATE | 5.00 |
| **C** | | | |
| Sodium Hydroxide, 10% | (1) | AQUA, SODIUM HYDROXIDE | 0.50 |

| **D** | | | |
|---|---|---|---|
| RonaFlair^{®} Boroneige^{®} SF-3 | (1) | BORON NITRIDE | 2.00 |

| **E** | | | |
|---|---|---|---|
| Preservatives | | | q.s. |

### Procedure:

Disperse Carbopol Ultrez 21 in the water and stir until homogeneous. Add the xanthan gum premixed with glycerin and stir until homogeneous. Add the active ingredients and stir until homogeneous. Add the Montanov and heat A to 80°C.

Prepare phase B and heat B to 80°C. Emulsion : pour B into A under high stirring. Neutralise with C and homogenize.

At T<60°C add D and stir until homogeneous. Slowly cool the cream under gentle stirring. At T<35°C add E and stir until homogeneous.
Notes: (1cP is 1mPa.s)
Appearance : thick white cream
pH : 6.3
Viscosity : 370 000 cP (Fungilab Expert, Spindle PD, 3 rpm). The product reaches its viscosity after 2 weeks
Stability : 3 months at RT, 4°C and 45°C
Suppliers:
   (1) Merck KGaA, Darmstadt, Germany / EMD Performance Materials
   (2) Gattefossé
   (3) Solvay
   (4) Seppic
   (5) BASF AG
   (6) Greentech SA
   (7) Croda

### Example 6: Day cream for sensitive and dry skin

| **Ingredients** | | **INCI (EU)** | **[%]** |
|---|---|---|---|
| **A** | | | |
| RonaCare^{®} Ectoin | (1) | ECTOIN | 1.00 |
| RonaCare^{®} Nicotinamide | (1) | NIACINAMIDE | 2.00 |
| Glycerol 85% | (1) | GLYCERIN, AQUA | 8.00 |
| Water, demineralized | | AQUA | ad 100 |

| **B** | | | |
|---|---|---|---|
| Montanov L | (2) | C14-22 ALCOHOLS, C12-20 ALKYL GLUCOSIDE | 3.00 |
| Cetiol SB 45 | (3) | BUTYROSPERMUM PARKII BUTTER | 3.00 |
| PARYOL 468 MG/R | (4) | MANGIFERA INDICA SEED BUTTER | 3.00 |
| Lanol 99 | (2) | ISONONYL ISONONANOATE | 10.00 |

| **C** | | | |
|---|---|---|---|
| RonaFlair^{®} White Sapphire | (1) | SYNTHETIC SAPPHIRE | 2.00 |

| **D** | | | |
|---|---|---|---|
| Sepinov EMT 10 | (2) | HYDROXYETHYL ACRYLATE, SODIUM ACRYLOYLDIMETHYL TAURATE COPOLYMER | 1.00 |

| **E** | | | |
|---|---|---|---|
| RonaCare^{®} VTA | (1) | AQUA, ALCOHOL, LECITHIN, DISODIUM RUTINYL DISULFATE, HYDROXYPROLINE, SORBITOL, MAGNESIUM ASCORBYL PHOSPHATE, TOCOPHEROL, | 4.00 |

| | | | |
|---|---|---|---|
| | | ASCORBYL PALMITATE, GLYCERYL STEARATE, GLYCERYL OLEATE, CITRIC ACID | |
| RonaCare^{®} Luremin^{®} | (1) | SORBITOL, DIHYDROXY METHYLCHROMONE | 2.00 |
| Water, demineralized | | AQUA | 10.00 |
| Preservatives | | | q.s. |

### Procedure:

Prepare phase A and mix it under stirring until homogeneous. Prepare B and heat A and B to 80°C. Add C to B and stir until homogeneous. Add D to B+C and stir until homogeneous .

Emulsion : pour B+C+D into A under stirring. Homogenize. Slowly cool the cream under gentle stirring.

At T<30°C add E premixed and stir until homogeneous.
Notes: (1cP is 1mPa.s)
Appearance : thick white cream
pH : 6.3
Viscosity : 120 000 cP (Fungilab Expert, Spindle PC, 5 rpm)
Stability : 3 months at RT, 4°C and 45°C
Suppliers:
   (1) Merck KGaA, Darmstadt, Germany / EMD Performance Materials
   (2) Seppic
   (3) BASF AG
   (4) Brenntag Chemiepartner GmbH

### Example 7: Night cream for sensitive and dry skin

| **Ingredients** | | **INCI (EU)** | **[%]** |
|---|---|---|---|
| A | | | |
| Oxynex^{®} 2004 | (1) | PROPYLENE GLYCOL, BHT, GLYCERYL STEARATE, GLYCERYL OLEATE, ASCORBYL PALMITATE, CITRIC ACID | 0.05 |
| Plurol diisostearique | (2) | POLYGLYCERYL-3 DIISOSTEARATE | 3.00 |
| Acticire | (2) | JOJOBA ESTERS, HELIANTHUS ANNUUS SEED CERA, ACACIA DECURRENS FLOWER CERA, POLYGLYCERIN-3 | 2.00 |
| Compritol 888 ATO | (2) | GLYCERYL BEHENATE | 1.00 |
| Cutina HR Powder | (3) | HYDROGENATED CASTOR OIL | 2.25 |
| Cetiol CC | (3) | DICAPRYLYL CARBONATE | 10.00 |
| Cetiol C5 | (3) | COCO CAPRYLATE | 5.00 |
| Dub PO | (4) | ETHYLHEXYL PALMITATE | 10.00 |

| **B1** | | | |
|---|---|---|---|
| RonaCare^{®} Ectoin | (1) | ECTOIN | 1.00 |
| RonaCare^{®} Luremin^{®} | (1) | SORBITOL, DIHYDROXY METHYLCHROMONE | 2.00 |
| RonaCare^{®} Magnesium Sulfate | (1) | MAGNESIUM SULFATE | 1.50 |
| RonaCare^{®} Sodium Chloride | (1) | SODIUM CHLORIDE | 1.50 |
| Water, demineralized | | AQUA | ad 100 |

| **B2** | | | |
|---|---|---|---|
| RonaCare^{®} Isoquercetin | (1) | ISOQUERCETIN | 0.10 |
| Glycerol 85% | (1) | GLYCERIN, AQUA | 8.00 |

| C | | | |
|---|---|---|---|
| RonaFlair^{®} Boroneige^{®} SF-3 | (1) | BORON NITRIDE | 2.00 |
| RonaFlair^{®} LDP White | (1) | SODIUM POTASSIUM ALUMINUM SILICATE, CI 77891, SILICA | 1.00 |
| RonaFlair^{®} Soft Sphere | (1) | SYNTHETIC FLUORPHLOGOPITE, SILICA | 2.00 |

| D | | | |
|---|---|---|---|
| RonaCare^{®} VTA | (1) | AQUA, ALCOHOL, LECITHIN, DISODIUM RUTINYL DISULFATE, HYDROXYPROLINE, SORBITOL, MAGNESIUM ASCORBYL PHOSPHATE, TOCOPHEROL, ASCORBYL PALMITATE, GLYCERYL STEARATE, GLYCERYL OLEATE, CITRIC ACID | 4.00 |
| Preservatives | | | q.s. |

### Procedure:

Heat phase A to 85°C under stirring. Heat phase B1 to 85°C under stirring.

Heat phase B2 to 85°C under stirring and check the dispersion of RonaCare^{®} Isoquercetin. Then add B2 to B1. Stir until homogeneous.

Emulsion : pour slowly B into A under rapid mixing and maintain rapid mixing for about 15 min.

After the emulsion step add slowly C and stir until homogeneous. Cool down and at T<40°C add slowly D under rapid mixing and stir until homogeneous.

Notes: (1cP is 1mPa.s)
Appearance : yellow cream
Viscosity : 85 000 cP (Fungilab Expert, Spindle PC, 5 rpm)
Stability : 3 months at RT, 4°C and 45°C
Suppliers:
   (1) Merck KGaA, Darmstadt, Germany / EMD Performance Materials
   (2) Gattefossé
   (3) BASF AG
   (4) Stearinerie Dubois Fils

## Claims

1. Non-therapeutic cosmetic use of at least one compatible solute selected from (S)-1,4,5,6-tetrahydro-2-methyl-4-pyrimidincarboxylic acid and (S,S)-1,4,5,6-tetrahydro-5-hydroxy-2-methyl-4-pyrimidincarboxylic acid for lightening of skin pores and/or for the reduction of the number of yellow or dark colored skin pores in human skin.

2. Non-therapeutic cosmetic use according to Claim 1 **characterized in that** the at least one compabtible solute is (S)-1,4,5,6-tetrahydro-2-methyl-4-pyrimidincarboxylic acid.

## Patentansprüche

1. Nicht therapeutische kosmetische Verwendung mindestens eines verträglichen gelösten Stoffs ausgewählt aus (S)-1,4,5,6-Tetrahydro-2-methyl-4-pyrimidincarbonsäure und (S,S)-1,4,5,6-Tetrahydro-5-hydroxy-2-methyl-4-pyrimidincarbonsäure zur Aufhellung von Hautporen und/oder zur Verringerung der Anzahl von gelb oder dunkel gefärbten Hautporen in menschlicher Haut.

2. Nicht therapeutische kosmetische Verwendung nach Anspruch 1, **dadurch gekennzeichnet, dass** es sich bei dem mindestens einen verträglichen gelösten Stoff um (S)-1,4,5,6-Tetrahydro-2-methyl-4-pyrimidincarbonsäure handelt.

## Revendications

1. Utilisation cosmétique non thérapeutique d'au moins un soluté compatible choisi parmi l'acide (S)-1,4,5,6-tétrahydro-2-méthyl-4-pyrimidinecarboxylique et l'acide (S,S)-1,4,5,6-tétrahydro-5-hydroxy-2-méthyl-4-pyrimidinecarboxylique pour l'éclaircissement de pores de la peau et/ou pour la réduction du nombre de pores de la peau colorés en jaune ou sombres dans la peau humaine.

2. Utilisation cosmétique non thérapeutique selon la revendication 1, **caractérisée en ce que** ledit au moins un soluté compatible est l'acide (S)-1,4,5,6-tétrahydro-2-méthyl-4-pyrimidinecarboxylique.
